# EUROPEAN PATENT APPLICATION

(11) **EP 4 810 622 A2**
(43) Date of publication of application: **23.09.2026**
(21) Application number: 26194809.5
(22) Date of filing: 21.12.2021
(51) Int. Cl.: C12Q 1/54

(54) **MODIFIED GLUCOSE DEHYDROGENASE**

(30) Priority: 21.12.2020 JP 2020211550
(62) Document number(s) of the earlier application(s) in accordance with Art. 76 EPC: 21910808.1 / 4 265 727
(71) Applicant: Amano Enzyme Inc., Nagoya-shi Aichi 460-8630 (JP); National University Corporation Tokyo University Of Agriculture and Technology, Fuchu-shi Tokyo 183-8538 (JP); The University of North Carolina at Chapel Hill, Chapel Hill, NC 27514 (US)
(72) Inventor: NISHIO, Kyoichi, Kakamigahara-shi, Gifu, 509-0109 (JP); TSUGAWA, Wakako, Fuchu-shi, Tokyo, 183-8538 (JP); SODE, Koji, Chapel Hill, North Carolina, 27516 (US)
(74) Representative: Müller-Boré & Partner Patentanwälte PartG mbB

(57) **Abstract**

The present invention relates to a modified glucose dehydrogenase. More specifically, the present invention relates to a polypeptide having an improved glucose dehydrogenase specific activity, a DNA encoding the polypeptide, a recombinant vector, a transformant, an enzyme agent, and a use application of the polypeptide.

## Description

### TECHNICAL FIELD

The present invention relates to a modified glucose dehydrogenase. More specifically, the present invention relates to a polypeptide having an improved glucose dehydrogenase specific activity, a DNA encoding the polypeptide, a recombinant vector, a transformant, an enzyme agent, and a use application of the polypeptide.

### BACKGROUND ART

Diabetics are increasing year by year, and diabetics, particularly insulin-dependent patients require daily monitoring of the blood glucose level for control of the blood glucose level. In recent years, a self blood glucose measuring instrument (glucose sensor), which uses an electrochemical biosensor capable of simply and accurately measuring the blood glucose level using an enzyme, has been widely used.

As the enzyme used in the glucose sensor, a glucose oxidase (hereinafter, also referred to as "GO") or a glucose dehydrogenase (hereinafter, also referred to as "GDH") using glucose as a substrate is used.

While GO has advantages of high specificity to glucose and excellent thermal stability, problems have been pointed out in that measurement using GO is susceptible to dissolved oxygen in a measurement sample, and dissolved oxygen affects the measurement result. In order to solve these problems, FAD-dependent GDH (hereinafter, referred to as "FAD-GDH") has been developed. However, since it is known that FAD-GDH reacts with xylose, in an examination for determining diabetes, not only oral glucose tolerance test, but also oral xylose tolerance test and intravenous xylose tolerance test are performed, and in view of this aspect, in the case of using FAD-GDH, a problem arises in that FAD-GDH affects the blood glucose level in the above tolerance test.

In this regard, in Patent Document 1, it has been reported that FAD-GDH obtained by mutating GO to GDH is obtained as an enzyme compensating for both defects of GO and GDH.

### PRIOR ART DOCUMENT

### PATENT DOCUMENT

Patent Document 1: WO 2011/068050 A

### SUMMARY OF THE INVENTION

### PROBLEMS TO BE SOLVED BY THE INVENTION

FAD-GDH obtained by mutating GO compensates for both defects of GO and GDH and is an enzyme excellent in substrate specificity, but there is room for improvement in specific activity thereof.

Therefore, an object of the present invention is to provide a glucose dehydrogenase having an improved specific activity.

### MEANS FOR SOLVING THE PROBLEM

The present inventors have conducted X-ray crystallography of FAD-GDH (SEQ ID NO: 1) in which GO is mutated, and as a result, have focused on the possibility that histidine at the 538th position is sterically hindered, and examined introduction of mutation to methionine at the 578th position near the histidine for the purpose of eliminating the steric hindrance at the site. As a result, present inventors have found that specific activity can be improved by introducing a predetermined mutation. The present invention has been completed based on this finding. That is, the present invention provides inventions of the following aspects.

Item 1. A polypeptide of any one of the following (1) to (3):
(1) a polypeptide comprising an amino acid sequence in which an amino acid residue at the 578th position is substituted with a valine residue or a phenylalanine residue in an amino acid sequence shown in SEQ ID NO: 1;
(2) a polypeptide comprising an amino acid sequence in which an amino acid residue at the 578th position is substituted with a valine residue or a phenylalanine residue in an amino acid sequence shown in SEQ ID NO: 1, and one or a few amino acid residues other than the amino acid residue introduced by the substitution are substituted, added, inserted, or deleted, and having an improved glucose dehydrogenase activity as compared with a polypeptide comprising an amino acid sequence shown in SEQ ID NO: 1; and
(3) a polypeptide comprising an amino acid sequence in which an amino acid residue at the 578th position is substituted with a valine residue or a phenylalanine residue in an amino acid sequence shown in SEQ ID NO: 1, having 70% or more sequence identity to an amino acid sequence shown in SEQ ID NO: 1 except for the amino acid residue introduced by the substitution, and having an improved glucose dehydrogenase activity as compared with a polypeptide comprising an amino acid sequence shown in SEQ ID NO: 1.

Item 2. A DNA encoding the polypeptide described in Item 1.

Item 3. An expression cassette or recombinant vector comprising the DNA described in Item 2.

Item 4. A transformant obtained by transforming a host with the expression cassette or recombinant vector described in Item 3.

Item 5. A method for producing the polypeptide described in Item 1, the method comprising a step of culturing the transformant described in Item 4.

Item 6. An enzyme agent comprising the polypeptide described in Item 1.

Item 7. A glucose measuring reagent comprising the polypeptide described in Item 1.

Item 8. A glucose measuring method comprising a step of measuring glucose in a sample using the polypeptide described in Item 1.

Item 9. A glucose measuring kit comprising the glucose measuring reagent described in Item 7.

Item 10. A glucose sensor comprising an insoluble support and the polypeptide described in Item 1 immobilized to the insoluble support.

Item 11. A glucose reducing agent comprising the polypeptide described in Item 1.

Item 12. A method for producing a composition having reduced glucose, the method comprising a step of reducing glucose in a composition selected from the group consisting of a food composition, a cosmetic composition, and a pharmaceutical composition, using the polypeptide described in Item 1.

### ADVANTAGES OF THE INVENTION

According to the present invention, there is provided a glucose dehydrogenase having an improved specific activity.

### BRIEF DESCRIPTION OF THE DRAWING

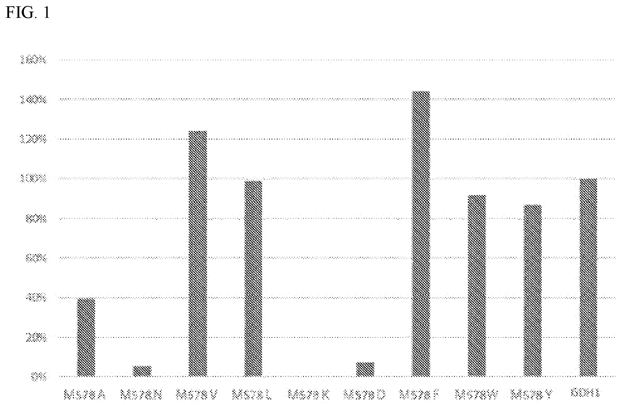

Fig. 1 shows the enzyme activity of a mutant at the 578th position of GDH comprising an amino acid sequence shown in SEQ ID NO: 1.

### EMBODIMENTS OF THE INVENTION

Hereinafter, the present invention will be described in detail. 20 amino acid residues in amino acid sequences excluding the sequence lists may be abbreviated as one-letter codes. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, treonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the present specification, the amino acid sequence to be displayed is N-terminal at the left end and C-terminal at the right end.

In the present specification, expression such as "A120G" is a notation of amino acid substitution. For example, "A120G" means that the 120th amino acid A from the N-terminal side in the specific amino acid sequence is substituted with an amino acid G.

In the present specification, the term "non-polar amino acid" includes alanine, valine, leucine, isoleucine, proline, methionine, phenylalanine, and tryptophan. The term "non-charged amino acid" includes glycine, serine, threonine, cysteine, tyrosine, asparagine, and glutamine. The term "acidic amino acid" includes aspartic acid and glutamic acid. The term "basic amino acid" includes lysine, arginine, and histidine.

In the present specification, the term "substitution" includes not only a case where a substitution of an amino acid residue is artificially introduced, but also a case where a substitution of an amino acid residue is naturally introduced, that is, a case where amino acid residues are originally different. In the present specification, the substitution of an amino acid residue may be an artificial substitution or a natural substitution, but an artificial substitution is preferred.

### 1. Polypeptide

A polypeptide of the present invention is a polypeptide of any one of the following (1) to (3):
(1) a polypeptide comprising an amino acid sequence in which an amino acid residue at the 578th position is substituted with a valine residue or a phenylalanine residue in an amino acid sequence shown in SEQ ID NO: 1;
(2) a polypeptide comprising an amino acid sequence in which an amino acid residue at the 578th position is substituted with a valine residue or a phenylalanine residue in an amino acid sequence shown in SEQ ID NO: 1, and one or a few amino acid residues other than the amino acid residue introduced by the substitution are substituted, added, inserted, or deleted, and having an improved glucose dehydrogenase activity as compared with a polypeptide comprising an amino acid sequence shown in SEQ ID NO: 1; and
(3) a polypeptide comprising an amino acid sequence in which an amino acid residue at the 578th position is substituted with a valine residue or a phenylalanine residue in an amino acid sequence shown in SEQ ID NO: 1, having 90% or more sequence identity to an amino acid sequence shown in SEQ ID NO: 1 except for the amino acid residue introduced by the substitution, and having an improved glucose dehydrogenase activity as compared with a polypeptide comprising an amino acid sequence shown in SEQ ID NO: 1.

The polypeptides shown in (1) to (3) have an improved glucose dehydrogenase activity as compared with a polypeptide of SEQ ID NO: 1.

The polypeptide of SEQ ID NO: 1 is D446H and V582P double mutants of glucose oxidase (GO) derived from Aspergillus niger (in the following, D446H and V582P are also described as "double mutations", separately from the amino acid substitution mutation at the 578th position described above).

The polypeptides of (1) to (3) include not only polypeptides obtained by artificial substitution but also polypeptides originally having such an amino acid sequence.

Hereinafter, in the polypeptides of (2) and (3), amino acid residues other than the amino acid residue at the 578th position of SEQ ID NO: 1 may be referred to as "arbitrary difference sites". In the present specification, the term "arbitrary difference site" is a site at which a difference is allowed as long as it does not significantly affect the properties of the polypeptide. In the present specification, a polypeptide having a difference in amino acid sequence at an arbitrary difference site as compared to the polypeptide of (1) but having an improved glucose dehydrogenase activity as compared with a polypeptide comprising an amino acid sequence shown in SEQ ID NO: 1 is referred to as a variant of the polypeptide of (1). The polypeptides of (2) and (3) are variants of the polypeptide of (1). It is more preferable that the variant of the polypeptide has a difference in the amino acid sequence at an arbitrary difference site as compared to the polypeptide of (1) but has substantially the same properties of the polypeptide. The expression "having substantially the same properties of the polypeptide" means having equivalent glucose dehydrogenase activity, and specifically means that the glucose dehydrogenase activity is about 0.8 to 1.2 times, preferably about 0.9 to 1.1 times, and more preferably about 0.95 to 1.05 times the glucose dehydrogenase activity of the polypeptide of (1).

The amino acid difference in the polypeptide of (2) may comprise only one of the differences (for example, substitution) including substitution, addition, insertion, and deletion or comprise two or more of the differences (for example, substitution and insertion). The number of amino acid differences at an arbitrary difference site in the polypeptide of (2) may be 1 or several, and is, for example, 1 to 60 or 1 to 50, preferably 1 to 20, 1 to 10, 1 to 8, 1 to 7, 1 to 6, 1 to 5, or 1 to 4, further preferably 1 to 3, and particularly preferably 1 or 2, or 1.

In the polypeptide of (3), the sequence identity to each amino acid sequence shown in SEQ ID NO: 1 except for the site at which the amino acid substitution has been made may be 70% or more, 80% or more, 85% or more, or 90% or more, but is preferably 92% or more or 94% or more, further preferably 95% or more, 96% or more, 97% or more, or 98% or more, even more preferably 99% or more, still more preferably 99.5% or more, and particularly preferably 99.8% or more.

In the polypeptide of (3), the sequence identity to each amino acid sequence shown in SEQ ID NO: 1 except for the site at which the amino acid substitution has been made is a sequence identity calculated by extracting only the arbitrary difference site from each amino acid sequence shown in SEQ ID NO: 1 and comparing only the arbitrary difference site. The "sequence identity" refers to a value of amino acid sequence identity obtained by bl2seq program (Tatiana A. Tatsusova, Thomas L. Madden, FEMS Microbiol. Lett., Vol. 174, p 247-250, 1999) in BLAST PACKAGE [sgi32 bit edition, Version 2.0.12; available from National Center for Biotechnology Information (NCBI)]. Parameter settings may be as follows: Gap insertion Cost value: 11 and Gap extension Cost value: 1.

When an amino acid substitution is introduced into the arbitrary difference sites of the polypeptides of (2) and (3), a conservative substitution is mentioned as an example of an aspect of the amino acid substitution. That is, examples of the amino acid substitution introduced into the arbitrary difference site in the polypeptides of (2) and (3) include the following substitutions: when an amino acid to be substituted is a non-polar amino acid, a substitution with other non-polar amino acids; when an amino acid to be substituted is a non-charged amino acid, a substitution with other non-charged amino acids; when an amino acid to be substituted is an acidic amino acid, a substitution with other acidic amino acids; and when an amino acid to be substituted is a basic amino acid, a substitution with other basic amino acids.

Other examples of the aspect of the amino acid substitution when an amino acid substitution is introduced into the arbitrary difference sites of the polypeptides of (2) and (3) include the following other predetermined substitutions. That is, since it is considered that one or two or more amino acid substitutions selected from the group consisting of the 115th position (L), the 131st position (G), the 132nd position (T), the 193rd position (V), the 353rd position (T), the 436th position (F), the 444th position (S), the 472nd position (Y), the 511th position (I), the 535th position (P), the 537th position (Y), and the 583rd position (M), and substitution of the 582nd position (P) with S, R, or L (hereinafter, these are referred to as "other predetermined substitutions") in the amino acid sequence shown in SEQ ID NO: 1 contribute to expression of the glucose dehydrogenase activity or expression of the glucose dehydrogenase activity and suppression of reactivity to xylose, the other substitutions may be introduced into the arbitrary difference sites of the polypeptides of (2) and (3). Preferred examples of the other predetermined substitutions include substitutions at the 446th position (H) and the 582nd position (P) with S, R, or L; and substitution of the 444th position (S) with R, Q, or E.

When the polypeptides of (2) and (3) do not include the other predetermined substitutions, since it is considered that the 446th position (H) and the 582nd position (P) in the amino acid sequence shown in SEQ ID NO: 1 contribute to expression of the glucose dehydrogenase activity and suppression of reactivity to xylose, it is desirable not to introduce substitutions or deletions at these sites.

When the polypeptides of (2) and (3) include the other predetermined substitutions, since it is considered that the other predetermined substitutions contribute to expression of the glucose dehydrogenase activity or expression of the glucose dehydrogenase activity and suppression of reactivity to xylose, a substitution or deletion may be introduced into any one or both of the 446th position (H) and the 582nd position (P) in the amino acid sequence shown in SEQ ID NO: 1. Specific examples of the polypeptides of (2) and (3) when having the other predetermined substitutions and having a substitution or deletion introduced into any one or both of the 446th position (H) and the 582nd position (P) include the following polypeptides of [a] and [b]:
[a] a polypeptide having features that in SEQ ID NO: 1, the 446th position (H) is not substituted; the 582nd position (P) is substituted with S, R, or L; and the 578th position (M) is substituted with V or P; and
[b] a polypeptide having features that in SEQ ID NO: 1, the 444th position (S) is substituted with R, Q, or E; the 446th position (H) is substituted with another amino acid (for example, D); the 582nd position (P) is not substituted; and the 578th position (M) is substituted with V or F.

In the polypeptides of (2) and (3), the degree of the glucose dehydrogenase activity of "a polypeptide having an improved glucose dehydrogenase activity as compared with a polypeptide comprising an amino acid sequence shown in SEQ ID NO: 1" is not particularly limited as long as it is higher than the glucose dehydrogenase activity of the polypeptide comprising an amino acid sequence shown in SEQ ID NO: 1, but the degree thereof is 1.10 times or more, preferably 1.15 times or more, and more preferably 1.20 times or more the glucose dehydrogenase activity of the polypeptide comprising an amino acid sequence shown in SEQ ID NO: 1. The upper limit of the degree of the glucose dehydrogenase activity of the polypeptides of (2) and (3) is not particularly limited, and is, for example, 1.40 times or less, 1.35 or less, or 1.30 or less the glucose dehydrogenase activity of the polypeptide comprising an amino acid sequence shown in SEQ ID NO: 1.

The polypeptide of the present invention catalyzes a reaction of oxidizing a hydroxyl group of glucose in the presence of an electron acceptor to produce glucono-δ-lactone. The "glucose dehydrogenase activity" of the polypeptide of the present invention can be measured using this working principle, for example, using the following system using, as electron acceptors, phenazine methosulfate (PMS) and 2,6-dichloroindophenol (DCIP).$\begin{matrix}\left(Reaction 1\right) & D - glucose + PMS \left(oxidized form\right) \\ & \to D - glucono - δ - lactone + PMS \left(reduced form\right) \\ \left(Reaction 2\right) & PMS \left(reduced form\right) + DCIP \left(oxidized form\right) \\ & \to PMS \left(oxidized form\right) + DCIP \left(reduced form\right)\end{matrix}$

In the above reaction 1, PMS (reduced form) is generated due to the oxidation of glucose. DCIP is reduced due to the oxidation of PMS (reduced form) by the above reaction 2 subsequently proceeding. The degree of disappearance of this "DCIP (oxidized form)" is sensed as the amount of change in absorbance at a wavelength of 600 nm, and the glucose dehydrogenase activity can be determined based on this amount of change.

Specifically, 2.05 mL of a 50 mmol/L phosphate buffer solution (pH 6.5), 0.60 mL of a 1 mol/L glucose solution, 0.10 mL of a 15 mmol/L PMS solution, and 0.15 mL of a 2 mmol/L DCIP solution are mixed, the mixture is incubated at 37°C for 5 minutes, 0.1 mL of an enzyme solution diluted with a 50 mmol/L phosphate buffer solution (pH 6.5) containing 0.1% BSA is then added thereto, and the reaction is started. A decrease in absorbance at 600 nm (ΔA600) per minute due to the progress of an enzyme reaction is determined and GDH activity is calculated according to the following formula. At this time, for GDH activity, the amount of an enzyme reducing 1 µmol of DCIP per minute in the presence of D-glucose is defined as 1 U.$\begin{matrix}GDH activity \\ \left(U/mL\right)\end{matrix} = \frac{- \left(ΔA600-ΔA600blank\right) \times Vt \times df}{16.3 \times 1.0 \times Vs}$

In the formula, Vt represents the total liquid amount (mL) of the reaction reagent solution and the enzyme solution, 16.3 represents the absorbance coefficient (cm²/µmol) per 1 µmol of the reduced DCIP under the present activity measurement conditions, 0.1 represents the liquid amount (mL) of the enzyme solution, 1.0 represents the optical path length (cm) of the cell, ΔA600blank represents the decrease in absorbance at 600 nm per minute in the case of adding the buffer solution used for diluting the enzyme instead of the enzyme solution to start the reaction, and df represents the dilution rate.

### 2.DNA

A DNA encoding the polypeptide of the present invention (hereinafter, also referred to as "DNA of the present invention") can be appropriately designed by a person skilled in the art according to the amino acid sequence of the polypeptide of the present invention. The DNA of the present invention may be obtained, for example, by introducing a mutation corresponding to the above-described amino acid mutation into a DNA encoding a polypeptide comprising an amino acid sequence shown in SEQ ID NO: 1, may be obtained by introducing a mutation corresponding to the above-described double mutation and the above-described amino acid mutation into a glucose oxidase (GO) derived from Aspergillus niger, that is, a DNA encoding a polypeptide in a form in which the above-described double mutation is not introduced into the amino acid sequence of SEQ ID NO: 1, or may be obtained by artificial synthesis based on a total gene synthesis method.

The DNA encoding a polypeptide comprising an amino acid sequence shown in SEQ ID NO: 1 is known as, for example, the nucleotide sequence shown in SEQ ID NO: 2, and for example, the nucleotide sequence site can be obtained from a nucleic acid construct such as a plasmid into which the nucleotide sequence is incorporated by a conventional method using PCR. A DNA encoding a glucose oxidase (GO) derived from A. niger is also known, and can be isolated from the genome DNA of A. niger by a conventional method using PCR.

The method for introducing a specific mutation into a specific site of a nucleotide sequence is well-known, and for example, a site-specific mutagenesis introduction method of DNA or the like can be used. Examples of a specific method of converting a base in a DNA include a method using a commercially available kit (such as QuickChange Lightning Site-Directed Mutagenesis kit: manufactured by Stratagene or KOD-Plus-Mutagenesis kit: manufactured by TOYOBO CO., LTD.).

The nucleotide sequence of the DNA into which a mutation has been introduced can be confirmed using a DNA sequencer. The nucleotide sequence is determined once, and thereafter, the DNA encoding the polypeptide can be obtained by chemical synthesis, PCR using a cloned probe as a template, or hybridization using a DNA fragment having the nucleotide sequence as a probe.

It is possible to synthesize a mutant form of DNA encoding the peptide, which has a function equivalent to that before mutation, by site-directed mutagenesis or the like. In order to introduce a mutation into the DNA encoding the peptide, a known method such as a Kunkel method, a Gapped duplex method, or a megaprimer PCR method can be used.

The DNA of the present invention is preferably a DNA in which the codon usage frequency is optimized for the host, and more preferably a DNA in which the codon usage frequency is optimized for a microorganism of the genus Aspergillus.

As an index representing the codon usage frequency, the total host optimal codon usage frequency of each codon may be adopted. The optimal codon is defined as a codon having the highest usage frequency among codons corresponding to the same amino acid. The codon usage frequency is not particularly limited as long as it is optimized for the host.

Examples of the DNA of the present invention include a DNA comprising a nucleotide sequence shown in SEQ ID NO: 3 or 4. The DNA comprising a nucleotide sequence shown in SEQ ID NO: 3 or 4 encodes a polypeptide in which the 578th position of the polypeptide of (1) is substituted with a valine residue or a phenylalanine residue.

Other examples of the DNA of the present invention include DNAs encoding a polypeptide having an improved glucose dehydrogenase activity as compared with a polypeptide comprising an amino acid sequence shown in SEQ ID NO: 1, and hybridizing, under stringent conditions, with a DNA comprising a nucleotide sequence that is complementary to a DNA comprising a nucleotide sequence shown in SEQ ID NO: 3 or 4.

Here, the expression "under stringent conditions" refers to conditions of incubating in 6×SSC (1×SSC is 0.15 M NaCl, 0.015 M sodium citrate, pH 7.0) containing 0.5% SDS, 5×Denhartz's [Denhartz's, 0.1% bovine serum albumin (BSA), 0.1% polyvinylpyrrolidone, 0.1% Ficoll 400], and 100 µg/ml of a salmon sperm DNA at 50°C to 65°C for 4 hours to overnight.

The hybridization under stringent conditions is specifically performed by the following method. That is, a nylon membrane on which a DNA library or a cDNA library is immobilized is prepared, and the nylon membrane is blocked at 65°C in a prehybridization solution containing 6×SSC, 0.5% SDS, 5×Denhartz's, and 100 µg/ml of a salmon sperm DNA. Thereafter, each probe labeled with ³²P is added and incubated at 65°C overnight. This nylon membrane is washed in 6×SSC at room temperature for 10 minutes, in 2×SSC containing 0.1% SDS at room temperature for 10 minutes, and in 0.2×SSC containing 0.1% SDS at 45°C for 30 minutes, then autoradiography is performed, and a DNA specifically hybridized with the probe can be detected.

Further examples of the DNA of the present invention include DNAs encoding a polypeptide having an improved glucose dehydrogenase activity as compared with a polypeptide comprising an amino acid sequence shown in SEQ ID NO: 1 and having 70% or more, 80% or more, 85% or more, or 90% or more homology of a DNA comprising a nucleotide sequence shown in SEQ ID NO: 3 or 4. The homology is preferably 92% or more or 94% or more, further preferably 95% or more, 96% or more, or 97% or more, even more preferably 98% or more or 99% or more, more preferably 99.5% or more, and particularly preferably 99.8% or more.

Here, the "homology" of DNA is calculated using a disclosed or commercially available software with an algorithm that makes a comparison using the standard sequence as a reference sequence. Specifically, BLAST, FASTA, GENETYX (manufactured by Software Development Co., Ltd), or the like can be used, and these may be used with default parameters being set.

### 3. Expression Cassette or Recombinant Vector

An expression cassette or recombinant vector containing the DNA encoding the polypeptide of the present invention (hereinafter, also referred to as "expression cassette of the present invention" or "recombinant vector of the present invention") can be obtained by linking a promoter and a terminator to the DNA of the present invention, or by inserting the expression cassette of the present invention or the DNA of the present invention into an expression vector.

The expression cassette or recombinant vector of the present invention may further include, as control factors, transcription elements such as an enhancer, a CCAAT box, a TATA box, and an SPI site, in addition to promoter and a terminator. These control factors may be operably linked to the DNA of the present invention. The expression "operably linked" means that various control factors that control the DNA of the present invention are linked to the DNA of the present invention in a state of being capable of operating in a host cell.

Regarding the recombinant vector of the present invention, as the expression vector, those constructed for recombination from phage, plasmid, or virus capable of autonomously growing in the host are suitable. Such expression vectors are well-known, and examples of a commercially available expression vector include pUC vector (Takara Bio Inc.), pQE vector (QIAGEN GmbH), pDR540 and pRIT2T (GE Healthcare Bio-Sciences KK), and pET vector (Merck KGaA, Darmstadt, Germany). The expression vector may be used by selecting an appropriate combination with a host cell.

### 4. Transformant

A transformant (hereinafter, also referred to as "transformant of the present invention" in some cases) is obtained by transforming a host with the expression cassette or recombinant vector of the present invention.

The host used for the production of the transformant is not particularly limited as long as the gene can be introduced, the expression cassette or recombinant vector is stable and capable of autonomous growth, and a trait of a gene comprising the DNA of the present invention can be expressed, but for example, microorganisms belonging to the genus Aspergillus such as Aspergillus oryzae, the genus of Escherichia such as Escherichia coli, the genus of Bacillus such as Bacillus subtilis, and the genus of Pseudomonas such as Pseudomonas putida; yeasts, and the like are preferably exemplified, and other than, the host may be an animal cells, an insect cell, a plant cell, and the like. Among these, a microorganism of the genus Aspergillus is particularly preferable.

The transformant of the present invention can be obtained by introducing the recombinant vector of the present invention into a host. The place where the DNA of the present invention is introduced is also not particularly limited as long as a target gene can be expressed, and may be on a plasmid or on a genome. Specific examples of the method for introducing the expression cassette of the present invention or the recombinant vector of the present invention include a recombinant vector method and a genome editing method. Conditions for introducing the expression cassette or the recombinant vector into the host may be appropriately set according to the type of the host, and the like. When the host is a microorganism, for example, a method using a competent cell by a calcium ion treatment, an electroporation method, a spheroplast method, and a lithium acetate method, and the like are mentioned. When the host is an animal cell, for example, an electroporation method, a calcium phosphate method, a lipofection method, and the like are mentioned. When the host is an insect cell, for example, a calcium phosphate method, a lipofection method, an electroporation method, and the like are mentioned. When the host is a plant cell, for example, an electroporation method, an Agrobacterium method, a particle gun method, a PEG method, and the like are mentioned.

Whether or not the expression cassette of the present invention or the recombinant vector of the present invention has been incorporated into a host can be confirmed by a PCR method, a Southern hybridization method, a Northern hybridization method, and the like.

When it is confirmed whether or not the expression cassette of the present invention or the recombinant vector of the present invention has been incorporated into a host by a PCR method, for example, the genome DNA, the expression cassette, or the recombinant vector may be separated and purified from a transformant.

For example, when the host is a bacterium, the separation and purification of the expression cassette or the recombinant vector are performed based on a lysate obtained by lysing the bacterium. As a lysis method, for example, a treatment is performed with a lytic enzyme such as lysozyme, and as necessary, a protease, other enzymes, and a surfactant such as sodium lauryl sulfate (SDS) are used in combination.

Physical crushing methods such as freeze thawing and French press processing may be combined. The separation and purification of a DNA from a lysate can be performed, for example, by appropriately combining a deproteinization treatment using a phenol treatment and a protease treatment, a ribonuclease treatment, an alcohol precipitation treatment, and a commercially available kit.

DNA cleavage can be performed according to a conventional method, for example, using a restriction enzyme treatment. As a restriction enzyme, for example, a type II restriction enzyme that acts on a specific nucleotide sequence is used. Binding between the DNA and the expression cassette or the expression vector is performed, for example, using a DNA ligase.

Thereafter, PCR is performed by designing a primer specific to the DNA of the present invention using the separated and purified DNA as a template. The amplification product obtained by PCR is subjected to agarose gel electrophoresis, polyacrylamide gel electrophoresis, capillary electrophoresis, or the like, and is stained with ethidium bromide, SYBR Green solution, and the like, and then the amplification product is detected as a band, so that transformation can be confirmed.

PCR can be performed using a primer labeled with a fluorescent dye or the like in advance to detect an amplification product. A method of binding an amplification product to a solid phase such as a microplate and confirming the amplification product by fluorescence, an enzyme reaction, or the like may also be adopted.

### 5. Method for Producing Polypeptide

The polypeptide of the present invention can be obtained by a production method including a step of culturing the transformant of the present invention.

The culture conditions of the transformant may be appropriately set in consideration of the nutritional physiological properties of the host, and liquid culture is preferably mentioned. In the case of performing industrial production, ventilating/stirring culture is preferred.

As a nutrient source of the medium, those required for growth of transformants can be used. A carbon source may be any assimilable carbon compound, and examples thereof include glucose, sucrose, lactose, maltose, molasses, and pyruvic acid.

A nitrogen source may be any assimilable nitrogen compound, and examples thereof include peptone, meat extract, yeast extract, casein hydrolysate, and soybean cake alkaline extract.

In addition to the carbon source and the nitrogen source, for example, phosphates, carbonates, sulfates, salts of magnesium, calcium, potassium, iron, manganese, zinc, and the like, specific amino acids, specific vitamins, and the like may be used as necessary.

The culture temperature can be appropriately set in a range in which the transformant of the present invention can grow and the transformant of the present invention produces the polypeptide of the present invention, but is preferably about 15 to 37°C. The culturing may be completed at an appropriate time with reference to the time when the polypeptide of the present invention reaches the highest yield, and the culturing time is usually about 12 to 48 hours.

The transformant of the present invention is cultured, the culture solution is collected by a method such as centrifugation to recover a supernatant or bacterial cells, the bacterial cells are treated by a mechanical method such as ultrasonic wave and French press or a lytic enzyme such as lysozyme, and as necessary, are solubilized by using an enzyme such as protease or a surfactant such as sodium lauryl sulfate (SDS), whereby a water-soluble fraction containing the polypeptide of the present invention can be obtained.

By selecting an appropriate expression vector and host, the expressed polypeptide of the present invention can also be secreted into the culture solution.

The water-soluble fraction containing the polypeptide of the present invention obtained as described above may be subjected to a purification treatment as it is, or may be subjected to a purification treatment after the polypeptide of the present invention in the water-soluble fraction is concentrated.

The concentration can be performed by, for example, concentration under reduced pressure, membrane concentration, a salting-out treatment, fractionation precipitation with a hydrophilic organic solvent (for example, methanol, ethanol, and acetone), or the like.

The purification treatment of the polypeptide of the present invention can be performed, for example, by appropriately combining methods such as gel filtration, adsorption chromatography, ion exchange chromatography, and affinity chromatography.

The purification treatment is already known, and can proceed by referring to appropriate documents, magazines, textbooks, and the like. The polypeptide of the present invention thus purified can be pulverized by freeze drying, vacuum drying, spray drying, or the like, as necessary, and then distributed to the market.

### 6. Enzyme Agent

The polypeptide of the present invention can be provided in a form of an enzyme agent. Therefore, the present invention also provides an enzyme agent containing the polypeptide of the present invention as an active ingredient.

The content of the polypeptide of the present invention in the enzyme agent of the present invention is not particularly limited, and can be appropriately set as long as the glucose dehydrogenase activity of the active ingredient is exhibited.

The enzyme agent of the present invention may contain other components to the extent that the effect of the present invention is not affected, in addition to the polypeptide of the present invention described above. Examples of the other components include enzymes other than the polypeptide of the present invention, additives, and culture residues generated by the production method.

The other enzymes can be appropriately determined according to the use application of the polypeptide of the present invention, and examples thereof include amylase (α-amylase, β-amylase, or glucoamylase), glucosidase (a-glucosidase or β-glucosidase), galactosidase (α-galactosidase or β-galactosidase), protease (acidic protease, neutral protease, or alkaline protease), peptidase (leucine peptidase or aminopeptidase), lipase, esterase, cellulase, phosphatase (acid phosphatase or alkaline phosphatase), nuclease, deaminase, oxidase, dehydrogenase (other than the above active ingredient), glutaminase, pectinase, catalase, dextranase, transglutaminase, protein deamidase, and pullulanase. These other enzymes may be contained singly or in combination of a plurality of kinds thereof.

The additive can be appropriately determined according to the use application of the polypeptide of the present invention and the formulation form of the enzyme agent, and examples thereof include an excipient, a buffer agent, a suspension agent, a stabilizer, a preservative, an antiseptic, and physiological saline. Examples of the excipient include starch, dextrin, maltose, trehalose, lactose, D-glucose, sorbitol, D-mannitol, sucrose, and glycerol. Examples of the buffer agent include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic include ethanol, benzalkonium chloride, paraoxybenzoic acid, and chlorobutanol. These additives may be contained singly or in combination of a plurality of kinds thereof.

Examples of the culture residues include components derived from a culture medium, contaminant proteins, and microbial cell components.

The formulation form of the enzyme agent of the present invention is not particularly limited, and examples thereof include a liquid form and a solid form (such as powder or granules). The enzyme agents in these formulation forms can be prepared by a generally known method.

Specific examples of the enzyme agent of the present invention include a glucose measuring reagent and a glucose reducing agent described below.

### 7. Use Application

The polypeptide of the present invention can be applied to use applications utilizing glucose dehydrogenase activity. Examples of such a use application include a use application for measuring glucose in a target sample and a use application for reducing glucose in a target composition.

When the polypeptide of the present invention is applied to a use application for measuring glucose in a target sample, more specifically, the polypeptide of the present invention can be used as an active ingredient of a glucose measuring reagent or as a recognition element of a glucose sensor. When the polypeptide of the present invention is applied to a use application for reducing glucose in a target composition, the polypeptide of the present invention can be used as an active ingredient of a glucose reducing agent.

That is, the present invention also provides a glucose measuring reagent, a glucose sensor, and a glucose reducing agent which contain the polypeptide of the present invention. Among these, other components that can be contained in addition to the active ingredient of the glucose measuring reagent and the glucose reducing agent and formulation forms thereof are the same as those described in the enzyme agent of the present invention, and specific use methods thereof will be specifically described below in "8. Glucose Measuring Method". The glucose sensor and a specific method for using the glucose sensor will be specifically described below in "10. Glucose Sensor".

### 8. Glucose Measuring Method

As described above, the polypeptide of the present invention can be used as a glucose measuring reagent. Therefore, the present invention also provides a glucose measuring method including a step of measuring glucose in a sample using the polypeptide of the present invention.

Examples of the sample include a sample for which a blood glucose level is to be measured (specifically, a blood sample), a sample for the amount of glucose in a food product is to be measured (specifically, seasoning, beverages, and the like), and a sample for which the degree of fermentation is to be measured in the production process of a fermented food product (specifically, vinegar, sake, and the like).

In the glucose measuring method of the present invention, the amount of glucose in a sample can be measured using an oxidation-reduction reaction by the polypeptide of the present invention. Specifically, an enzyme reaction is performed by bringing a sample containing glucose into contact with the polypeptide of the present invention, and the amount of reacted glucose can be measured based on a signal change for monitoring the enzymatic reaction.

More specifically, the polypeptide of the present invention can be brought into contact with the sample containing glucose together with an electron acceptor (such as 2,6-dichlorophenolindophenol (DCPIP) or phenazine methosulfate (PMS)), and a reaction promoter (for example, N-(2-acetamide)imide diacetic acid (ADA), bis(2-hydroxyethyl)imino tris(hydroxymethyl)methane (Bis-Tris), sodium carbonate, imidazole, or the like), a pH buffer agent, and/or a coloring reagent used as necessary, and reacted for a certain period of time. During this time, a reduction change of the electron acceptor, a signal change based on polymerization generation of a dye by receiving an electron from the electron acceptor (for example, absorbance change), or the like can be monitored. The glucose concentration in the sample can be calculated based on a calibration curve created in advance using a glucose solution having a standard concentration from a signal change until the time point when all the glucose in the sample is oxidized.

The conditions in the above enzyme reaction are appropriately determined based on the optimum temperature, the optimum pH, and the like of the polypeptide of the present invention.

### 9. Glucose Measuring Kit

The present invention also provides a glucose measuring kit used for performing the glucose measuring method. The glucose measuring kit of the present invention contains the glucose measuring reagent described above in "7. Use Application".

The glucose measuring kit of the present invention can contain, as items other than the glucose measuring reagent, a reagent, such as a buffer agent, an electron acceptor, a reaction promoter, and/or a coloring reagent, necessary for assay, a glucose standard solution for preparing a calibration curve, and the like. Specific examples and methods of use of these other items are as described above in "8. Glucose Measuring Method". The glucose measuring kit of the present invention may contain these other items singly or in combination of two or more kinds thereof.

### 10. Glucose Sensor

As described above, the polypeptide of the present invention can be used as a recognition element of a glucose sensor. Therefore, the present invention also provides a glucose sensor including an insoluble support and the polypeptide of the present invention immobilized to the insoluble support.

The immobilization mode of the polypeptide is not particularly limited, and examples thereof include physical immobilization by adhesion, adsorption, absorption, encapsulation, swelling, and the like, chemical or biochemical immobilization by specific non-covalent bonding, and chemical immobilization by covalent bonding.

In the polypeptide, the polypeptide may be immobilized on the insoluble support in a single form, or the polypeptide may be immobilized on the insoluble support in a form of a composition together with other components. A specific aspect when the polypeptide is immobilized on the insoluble support in a form of a composition includes an aspect in which the composition is physically immobilized on the insoluble support.

At least the surface of the insoluble support is composed of a solid substance or a solid material which does not dissolve in the reaction system between the polypeptide of the present invention and glucose and can immobilize the polypeptide. Specific examples of the material constituting at least the surface of the insoluble support include a swellable material (for example, a polymer matrix such as gelatin), a porous substrate (for example, a porous body such as cellulose, or a nonwoven sheet such as filter paper), a resin, glass, a redox polymer (that is, a polymer having a redox mediator bonded thereto), a metal, and carbon.

The shape of an insoluble carrier is not particularly limited, and examples thereof include a substrate shape, a flake shape, a stick shape, and a bead shape.

Preferred examples of the insoluble carrier include an electrode whose surface is composed of the above material (for example, a gold electrode, a platinum electrode, a carbon electrode, or the like). The polypeptide is preferably physically immobilized on such an electrode, and more preferably, the polypeptide is physically immobilized in a form of a composition containing other components (for example, mediators such as ferrocene and ferricyanide, phenazine methosulfate, and the like) together.

A specific aspect of the glucose sensor of the present invention may be any form employed as a biosensor, and examples thereof include a sensor chip, a microtiter plate, a test strip, and an electrochemical flow cell.

The sensing of glucose in such a specific embodiment can be performed by a method based on the method described above in "8. Glucose Measuring Method", particularly by the following method. A buffer solution is placed in a thermostatic cell and maintained at a constant temperature. As a working electrode, an electrode on which the polypeptide of the present invention is immobilized is used, and a counter electrode (for example, a platinum electrode) and a reference electrode (for example, an Ag/AgCl electrode) are used. After a constant voltage is applied to the electrode and the current becomes steady, a sample containing glucose (preferably, a blood sample, more preferably, autologous blood) is brought into contact with the electrode to measure an increase in current. The glucose concentration in the sample can be calculated according to the calibration curve created by a glucose solution having a standard concentration.

### 11. Method for Producing Composition Having Reduced Glucose

As described above, the polypeptide of the present invention can be used as a glucose reducing agent. Therefore, the present invention also provides a method for producing a composition having reduced glucose, the method including a step of reducing glucose in a composition selected from the group consisting of a food composition, a cosmetic composition, and a pharmaceutical composition, using the polypeptide of the present invention.

In the production method of the present invention, glucose in the composition is converted into glucono-δ-lactone using an oxidation-reduction reaction by the polypeptide of the present invention, so that the amount of glucose can be reduced. Specifically, the enzyme reaction may be performed by bringing the composition containing glucose into contact with the polypeptide of the present invention. The conditions in the enzyme reaction are appropriately determined based on the optimum temperature, the optimum pH, and the like of the polypeptide of the present invention.

A food composition, a cosmetic composition, and a pharmaceutical composition having reduced glucose, which are obtained by the production method of the present invention, can suppress coloring due to the Maillard reaction.

### EXAMPLES

Hereinafter, the present invention will be specifically described by means of Examples; however, the present invention is not to be construed as being limited to the following Examples.

### Test Example 1

As a result of X-ray crystallography of a glucose dehydrogenase comprising an amino acid sequence of SEQ ID NO: 1 (D446H and V582P double mutants of a glucose oxidase derived from Aspergillus niger; hereinafter, also described as "GDH1"), the present inventors have focused on the possibility that histidine at the 538th position was sterically hindered due to introduction of mutation to the amino acid at the 582nd position. For the purpose of eliminating this the steric hindrance, introduction of mutation to methionine at the 578th position near histidine at the 538th position was examined.

Based on the nucleotide sequence shown in SEQ ID NO: 2 encoding the amino acid sequence of SEQ ID NO: 1, the following primers for mutation were designed.
Primer 1 for M578A mutation:
   ACGCAAGCCTCGTCCCATCCTATGACG (SEQ ID NO: 5)
Primer 2 for M578A mutation:
   GGACGAGGCTTGCGTAGGGGGAATAGA (SEQ ID NO: 6)
Primer 1 for M578N mutation:
   ACGCAAAACTCGTCCCATCCTATGACG (SEQ ID NO: 7)
Primer 2 for M578N mutation:
   GGACGAGTTTTGCGTAGGGGGAATAGA (SEQ ID NO: 8)
Primer 1 for M578V mutation:
   ACGCAAGTGTCGTCCCATCCTATGACG (SEQ ID NO: 9)
Primer 2 for M578V mutation:
   GGACGACACTTGCGTAGGGGGAATAGA (SEQ ID NO: 10)
Primer 1 for M578L mutation: TACGCAACTGTCGTCCCATCCTATG (SEQ ID NO: 11)
Primer 2 for M578L mutation: GACGACAGTTGCGTAGGGGGAATAG (SEQ ID NO: 12)
Primer 1 for M578K mutation:
   ACGCAAAAGTCGTCCCATCCTATGACG (SEQ ID NO: 13)
Primer 2 for M578K mutation:
   GGACGACTTTTGCGTAGGGGGAATAGA (SEQ ID NO: 14)
Primer 1 for M578D mutation:
   ACGCAAGATTCGTCCCATCCTATGACG (SEQ ID NO: 15)
Primer 2 for M578D mutation:
   GGACGAATCTTGCGTAGGGGGAATAGA (SEQ ID NO: 16)
Primer 1 for M578F mutation:
   ACGCAATTCTCGTCCCATCCTATGACG (SEQ ID NO: 17)
Primer 2 for M578F mutation:
   GGACGAGAATTGCGTAGGGGGAATAGA (SEQ ID NO: 18)
Primer 1 for M578W mutation:
   ACGCAATGGTCGTCCCATCCTATGACG (SEQ ID NO: 19)
Primer 2 for M578W mutation:
   GGACGACCATTGCGTAGGGGGAATAGA (SEQ ID NO: 20)
Primer 1 for M578Y mutation:
   ACGCAATACTCGTCCCATCCTATGACG (SEQ ID NO: 21)
Primer 2 for M578Y mutation:
   GGACGAGTATTGCGTAGGGGGAATAGA (SEQ ID NO: 22)

PCR was performed using the plasmid inserted into the DNA comprising a nucleotide sequence of SEQ ID NO: 2 as a template and using the designed primer and DNA Polymerase to obtain an amplified DNA fragment.

The amplification product after PCR was treated with restriction enzyme Dpn I, and the unmutated plasmid was digested, and then ligated to obtain a mutation-introduced plasmid. The plasmid obtained after mutation introduction was transformed into E. coli DH5α, and then subjected to plasmid extraction to produce a mutation-introduced plasmid. The obtained mutation-introduced plasmid was transformed into Saccharomyces cerevisiae. The obtained transformed strain was liquid-cultured, and the enzyme solution thus obtained was examined for GDH activity by the following method.

### GDH Activity Evaluation Method

2.05 mL of a 50 mmol/L phosphate buffer solution (pH 6.5), 0.60 mL of a 1 mol/L glucose solution, 0.10 mL of a 15 mmol/L PMS solution, and 0.15 mL of a 2 mmol/L DCIP solution were mixed, the mixture was incubated at 37°C for 5 minutes, 0.1 mL of an enzyme solution diluted with a 50 mmol/L phosphate buffer solution (pH 6.5) containing 0.1% BSA was then added thereto, and the reaction was started. A decrease in absorbance at 600 nm (ΔA600) per minute due to the progress of an enzyme reaction was determined and GDH activity was calculated according to the following formula. At this time, for GDH activity, the amount of an enzyme reducing 1 µmol of DCIP per minute in the presence of D-glucose was defined as 1 U.$\begin{matrix}GDH activity \\ \left(U/mL\right)\end{matrix} = \frac{- \left(ΔA600-ΔA600blank\right) \times Vt \times df}{16.3 \times 1.0 \times Vs}$

In the formula, Vt represents the total liquid amount (mL) of the reaction reagent solution and the enzyme solution, 16.3 represents the absorbance coefficient (cm²/µmol) per 1 µmol of the reduced DCIP under the present activity measurement conditions, 0.1 represents the liquid amount (mL) of the enzyme solution, 1.0 represents the optical path length (cm) of the cell, ΔA600blank represents the decrease in absorbance at 600 nm per minute in the case of adding the buffer solution used for diluting the enzyme instead of the enzyme solution to start the reaction, and df represents the dilution rate.

The GDH activity of the enzyme GDH1 before mutation introduction was defined as 100%, and the relative amount (%) of the GDH activity of each mutant enzyme was calculated. The results are shown in Fig. 1. As shown in Fig. 1, improvement in activity was recognized in M578V and M578F. The expression amount of each enzyme was evaluated by SDS-PAGE, and it was confirmed that the expression amount was at an equivalent level. Therefore, it was recognized that the specific activity was improved in M578V and M578F.

### Test Example 2

The GDH gene part was amplified by PCR from the GDH1 plasmid in which mutation of M578V had been introduced, and the amplified DNA fragment was connected between the Taka-amylase-modified CS3 promoter and terminator genes of Aspergillus oryzae-derived FAD-dependent glucose dehydrogenase to construct an expression cassette.

The constructed expression cassette and the Aspergillus oryzae-derived orotidine 5'-phosphoric acid decarboxylase gene (pyrG gene) were inserted into pUC19 to construct an expression plasmid. The pyrG gene-defect strain of Aspergillus oryzae RIB40 was transformed using the constructed expression plasmid, and a transformed strain was obtained using uridine auxotrophy. The obtained transformed strain was liquid-cultured to obtain a crude enzyme liquid containing M578V. The obtained crude enzyme liquid was purified using salting out, hydrophobic bond chromatography, and ion exchange chromatography, and freeze-dried to obtain a purified enzyme powder of M578V.

The GDH activity value (U/mg) per unit weight of the obtained purified enzyme powder of M578V and purified enzyme powder of GDH1 was measured in the same manner as in Test Example 1, the relative amount (relative activity) of GDH activity of M578V was calculated, and the relative activity of M578V when the activity value of GDH1 was taken as 100% was derived. The results are shown in Table 1.

**[Table 1]**

| | Relative activity |
|---|---|
| GDH1 | 100% |
| M578V | 122% |

As shown in Table 1, M578V showed about 20% improvement in specific activity as compared to GDH1.

Kinetic parameters (Km [mmol/L] and Vmax [U/mg]) for glucose were determined, and the relative Km and relative Vmax relative activities of M578V when Km and Vmax of GDH1 were taken as 100%, respectively, were derived. The results are shown in Table 2.

**[Table 2]**

| | Relative Km | Relative Vmax |
|---|---|---|
| GDH1 | 100% | 100% |
| M578V | 103% | 127% |

As shown in Table 2, M578V showed almost no difference in Km for glucose as compared to GDH1, while Vmax showed about 20% improvement. That is, it was recognized that M578V showed almost no difference in enzyme affinity to a glucose substrate as compared to GDH1, but the efficiency of the enzyme reaction was improved.

SEQ ID NO: 1 is D446H and V582P double mutants of a glucose oxidase derived from Aspergillus niger.

SEQ ID NO: 2 is a DNA encoding D446H and V582P double mutants of a glucose oxidase derived from Aspergillus niger.

SEQ ID NO: 3 is a DNA encoding an M578V mutant of the double mutant shown in SEQ ID NO: 1.

SEQ ID NO: 4 is a DNA encoding an M578F mutant of the double mutant shown in SEQ ID NO: 1.

SEQ ID NOs: 5 to 22 are primers for mutation introduction.

The present invention relates to the following items:
1. A polypeptide of any one of the following (1) to (3):
   (1) a polypeptide comprising an amino acid sequence in which an amino acid residue at the 578th position is substituted with a valine residue or a phenylalanine residue in an amino acid sequence shown in SEQ ID NO: 1;
   (2) a polypeptide comprising an amino acid sequence in which an amino acid residue at the 578th position is substituted with a valine residue or a phenylalanine residue in an amino acid sequence shown in SEQ ID NO: 1, and one or a few amino acid residues other than the amino acid residue introduced by the substitution are substituted, added, inserted, or deleted, and having an improved glucose dehydrogenase activity as compared with a polypeptide comprising an amino acid sequence shown in SEQ ID NO: 1; and
   (3) a polypeptide comprising an amino acid sequence in which an amino acid residue at the 578th position is substituted with a valine residue or a phenylalanine residue in an amino acid sequence shown in SEQ ID NO: 1, having 70% or more sequence identity to an amino acid sequence shown in SEQ ID NO: 1 except for the amino acid residue introduced by the substitution, and having an improved glucose dehydrogenase activity as compared with a polypeptide comprising an amino acid sequence shown in SEQ ID NO: 1.
2. A DNA encoding the polypeptide according to item 1.
3. An expression cassette or recombinant vector comprising the DNA according to item 2.
4. A transformant obtained by transforming a host with the expression cassette or recombinant vector according to item 3.
5. A method for producing the polypeptide according to item 1, the method comprising a step of culturing the transformant according to item 4.
6. An enzyme agent comprising the polypeptide according to item 1.
7. A glucose measuring reagent comprising the polypeptide according to item 1.
8. A glucose measuring method comprising a step of measuring glucose in a sample using the polypeptide according to item 1.
9. A glucose measuring kit comprising the glucose measuring reagent according to item 7.
10. A glucose sensor comprising an insoluble support and the polypeptide according to item 1 immobilized to the insoluble support.
11. A glucose reducing agent comprising the polypeptide according to item 1.
12. A method for producing a composition having reduced glucose, the method comprising a step of reducing glucose in a composition selected from the group consisting of a food composition, a cosmetic composition, and a pharmaceutical composition, using the polypeptide according to item 1.

## Claims

1. A polypeptide of any one of the following (a1) to (a3):
(a1) a polypeptide comprising an amino acid sequence in which the amino acid residue at the 578th position is substituted with a valine residue in the amino acid sequence shown in SEQ ID NO: 1;
(a2) a polypeptide comprising an amino acid sequence in which the amino acid residue at the 578th position is substituted with a valine residue in the amino acid sequence shown in SEQ ID NO: 1, and 1 to 20 amino acid residue(s) other than the amino acid residue introduced by the substitution are substituted, added, inserted, or deleted, and having an improved glucose dehydrogenase activity as compared with a polypeptide comprising the amino acid sequence shown in SEQ ID NO: 1;
(a3) a polypeptide comprising an amino acid sequence in which the amino acid residue at the 578th position is substituted with a valine residue in the amino acid sequence shown in SEQ ID NO: 1, having 97% or more sequence identity to an amino acid sequence shown in SEQ ID NO: 1 except for the amino acid residue introduced by the substitution, and having an improved glucose dehydrogenase activity as compared with a polypeptide comprising the amino acid sequence shown in SEQ ID NO: 1.

2. A DNA encoding the polypeptide according to claim 1.

3. An expression cassette or recombinant vector comprising the DNA according to claim 2.

4. A transformant obtained by transforming a host with the expression cassette or recombinant vector according to claim 3.

5. A method for producing the polypeptide according to claim 1, the method comprising a step of culturing the transformant according to claim 4.

6. An enzyme agent comprising the polypeptide according to claim 1.

7. A glucose measuring reagent comprising the polypeptide according to claim 1.

8. A glucose measuring method comprising a step of measuring glucose in a sample using the polypeptide according to claim 1.

9. A glucose measuring kit comprising the glucose measuring reagent according to claim 7.

10. A glucose sensor comprising an insoluble support and the polypeptide according to claim 1 immobilized to the insoluble support.

11. A glucose reducing agent comprising the polypeptide according to claim 1.

12. A method for producing a composition having reduced glucose, the method comprising a step of reducing glucose in a composition selected from the group consisting of a food composition, a cosmetic composition, and a pharmaceutical composition, using the polypeptide according to claim 1.
